# EUROPEAN PATENT APPLICATION

(11) **EP 1 795 579 A1**
(43) Date of publication of application: **13.06.2007**
(21) Application number: 06256029.7
(22) Date of filing: 24.11.2006
(51) Int. Cl.: C10L 1/14, C10L 1/22, C09K 8/524, C07D 233/00

(54) **Asphaltene dispersants for petroleum products**

(30) Priority: 07.12.2005 US 748053
(71) Applicant: Rohm and Haas Company, Philadelphia, PA 19106-2399 (US)
(72) Inventor: Chheda, Bharati Dinkar, Houston, Texas 77068 (US)
(74) Representative: Kent, Venetia Katherine

(57) **Abstract**

An imidazoline-containing product useful for dispersing asphaltenes in heavy crude oil or residual fuel oil.

## Description

### Background

This invention relates generally to a composition useful in processing and handling petroleum products containing asphaltenes, including crude oil.

Certain petroleum products, including, e.g., heavy crude oils and residual fuel oils, which include materials referred to as "tars," "petroleum tars" or "tar sands," are rich in asphaltenes, metals and resins. The presence of these types of compounds can lead to various problems in the recovery, transportation, treatment and refining of petroleum hydrocarbons, including increased viscosity, formation of stable emulsions, fouling and corrosion. Residual fuel oils are prone to formation of asphaltene-containing precipitates during storage, or when fuel oils from different sources are mixed, especially when one of the oils has a high paraffin content. U.S. Pat. No. 6,402,934 discloses stabilization of asphaltenes in petroleum products using amine-chelate complexes. However, there is a need for improved additives to further enhance stabilization of asphaltenes.

The problem addressed by this invention is to find an improved composition suitable for stabilization of asphaltenes in petroleum products containing asphaltenes.

### Statement of Invention

The present invention is directed to a composition comprising a reaction product of: (i) an imidazoline compound having two C₉-C₂₁ alkyl or C₉-C₂₁ alkenyl groups and (ii) an organic carboxylic acid having at least two carbon atoms and having at least one hydroxy group or at least one additional carboxyl group.

The present invention is further directed to a method for dispersing asphaltenes in heavy crude oil or residual fuel oil; said method comprising adding to said heavy crude oil or residual fuel oil a composition comprising a reaction product of: (i) an imidazoline compound having two C₉-C₂₁ alkyl or C₉-C₂₁ alkenyl groups and (ii) an organic carboxylic acid.

### Detailed Description

All percentages are weight percentages, unless otherwise indicated. An "alkyl" group is a hydrocarbyl group having from one to twenty-two carbon atoms in a linear, branched or cyclic arrangement. Substitution on alkyl groups of one or more of halo, cyano, alkyl, or alkoxy is permitted; alkoxy groups may in turn be substituted by one or more halo substituents. Preferably, alkyl groups are unsubstituted. An "alkenyl" group is an alkyl group having at least one carbon-carbon double bond. An "organic carboxylic acid" is a compound having from one to twenty-two carbon atoms and at least one carboxyl group. An "imidazoline compound" is one containing an imidazoline ring, 4,5-dihydro-1H-imidazole.

In one embodiment of the invention, the organic carboxylic acid has from two to twenty-two carbon atoms, alternatively from two to ten carbon atoms. Preferred organic carboxylic acids include, e.g., tartaric acid, oxalic acid, citric acid, ascorbic acid, malic acid, malonic acid, lactic acid, gluconic acid, salicylic acid, succinamic acid and succinic acid. In one embodiment of the invention, the organic carboxylic acid is a chelating aminocarboxylic acid, i.e., a compound having an amine group, and having at least two carboxylic acid groups that can form coordinate bonds to a single metal atom. Preferred chelating aminocarboxylic acids useful in the present invention include, e.g., ethylenediaminetetraacetic acid (EDTA), hydroxyethylethylenediaminetriacetic acid, nitrilotriacetic acid (NTA), N-dihydroxyethylglycine and ethylenebishydroxyphenyglycine. Particularly preferred chelating aminocarboxylic acids are EDTA and NTA, and EDTA is most preferred.

In one embodiment of the invention, a C₉-C₂₁ alkyl or C₉-C₂₁ alkenyl group on an imidazoline is a C₁₅-C₂₁ alkyl or C₁₅-C₂₁ alkenyl group, or a mixture thereof; alternatively C₁₅-C₁₇ alkyl, C₁₅-C₁₇ alkenyl, or a mixture thereof; alternatively acyclic linear C₁₅-C₁₇ alkyl and/or C₁₅-C₁₇ alkenyl, including, but not limited to the alkenyl group of oleic acid (heptadec-8(Z)-ene-1-yl), n-heptadecyl, and mixtures of C₁₅-C₁₇ acyclic linear alkyl and/or alkenyl groups, e.g., those alkyl/alkenyl mixtures existing in naturally-occurring C₁₆-C₁₈ fatty acids.

In one embodiment, the imidazoline compound is a reaction product of a fatty acid and a polyamine. Preferred polyamines include, e.g., ethylenediamine, diethylenetriamine, and hydroxyethyl ethylenediamine..Fatty acids include, e.g., C₁₂-C₂₀ alkyl and/or alkenyl carboxylic acids, including polyunsaturated acids. Preferred fatty acids include oleic, linoleic and fatty acid mixtures derived from tall oil, soybean or palm oils. Preparation of fatty acid-polyamine reaction products is known, and is disclosed, e.g., in WO 01/25214.

In one preferred embodiment in which the imidazoline compound is a reaction product of a C₁₆-C₂₂ fatty acid and a polyamine in a 2:1 mole ratio, and the polyamine is diethylenetriamine, the imidazoline has the following structure 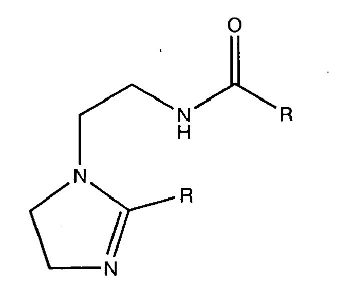 wherein R is a C₁₅-C₂₁ alkyl or alkenyl group derived from a C₁₆-C₂₂ carboxylic acid. The two "R" groups may be different when the imidazoline is derived from a mixture of carboxylic acids. When the imidazoline is derived from oleic acid and diethylenetriamine, it has the following structure 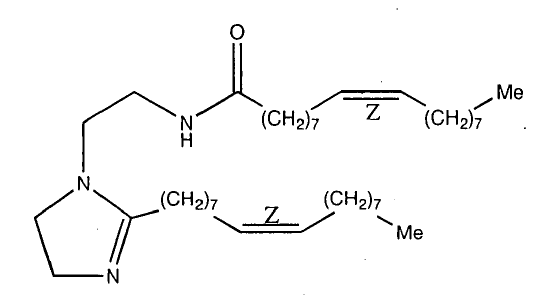 C₄₀H₇₅N₃O
9-Octadecenamide,
N-[2-[2-(8Z)-8-heptadecenyl-4,5-dihydro-1H-imidazol-1-yl]-, (9Z)-(9Cl)

In one embodiment of the invention, the imidazoline-organic carboxylic acid reaction product comprises the organic carboxylic acid and the imidazoline compound in a mole ratio from 30:1 to 1:30, respectively, alternatively from 20:1 to 1:20, respectively; alternatively from 10:1 to 1:10, respectively; alternatively from 10:1 to 1:5, respectively. Typically, the reaction product is formed by heating the imidazoline and the carboxylic acid, preferably at a temperature from 65 °C to 150 °C, preferably for 1 to 4 hours.

In one embodiment of the invention, the organic carboxylic acid and the imidazoline compound are present in the petroleum product in a total amount from 0.01 ppm to 500 ppm, alternatively from 0.02 ppm to 250 ppm, alternatively from 0.05 ppm to 100 ppm, alternatively from 0.05 ppm to 25 ppm.

For the purposes of this invention petroleum products containing asphaltenes can be any crude or refined product derived from petroleum, wherein the product contains asphaltenes. Preferably, the petroleum product containing asphaltenes is heavy crude oil or residual fuel oil. Residual fuel oil is the heavier fuel oil that remains after distilling light hydrocarbons and distillate fuel oils in refinery operations. Residual fuel oils typically are designated as number 5 or number 6 fuel oil, and conform to ASTM Specifications D 396 and D 975, and Federal Specification W-F-815C. Number 6 fuel oil for marine applications is also known as Bunker C fuel oil.

In addition to dispersing asphaltenes, the composition of the present invention typically also increases demulsibility, reduces viscosity, reduces sediment formation, reduces surface fouling and reduces corrosion. For crude oil recovery, the composition of the present invention can be injected directly into an injection well, or preferably diluted with solvent prior to injection. Suitable solvents include but are not limited to: petroleum distillates such as kerosene and gas oil; linear and branched aliphatic solvents such as pentane, hexanes, mixtures of nonanes and 2-ethylhexanes; cycloaliphatic mixtures commonly known as naphtha; aromatic solvents such as toluene, xylenes and commercial aromatic solvent mixtures; esters; ethers; alcohols such as ethanol, isopropanol, octanol and dodecanol; ketones such as acetone, cyclohexanone and acetophenone; and other polar solvents. Two preferred solvents are AROMATIC 150 solvent, a mixture with a boiling range of 184-204°C which contains xylene isomers; and AROMATIC 100 solvent, a mixture with a boiling range of 160-171°C which comprises >99% of various aromatic hydrocarbons, both of which are available from Exxon Mobil Chemical Co., Houston TX. Preferred dilutions are 0.01 to 50 wt% of the compound in the solvent, more preferred dilutions being 0.01 to 20 wt%, more preferred dilutions being 0.1 to 10%, and most preferred dilutions being 1 to 10 wt %.

### Examples

### General Procedure for Forming Imidazoline- or Amine-Acid Reaction Products:

To a 3-necked round bottom flask equipped with a mechanical stirrer, heating mantle, addition funnel and reflux condenser was added the imidazoline or amine and the contents were heated to 60 °C with stirring. The carboxylic acid was added, and the reaction temperature was maintained at 100 °C for about 2 hours, or until the reaction mixture was free of solids and haze. The mixture was cooled, and AROMATIC 150 solvent was added. The total amount of the imidazoline or amine plus the carboxylic acid was 10% of the total mixture, with the solvent being 90%. Relative amounts of reactants were calculated from the mole ratios indicated in Table 1, below.

### Asphaltene Dispersancy Test Methods:

This test requires a previously made dispersion of asphaltene in xylenes (Aromatic 150 solvent) or asphaltenic heavy crude diluted in xylenes (Aromatic 150 solvent) at a known concentration. A solution of an additive formulation (0.1 mL, the active ingredient was typically at 5-10 wt%, making the treat rate 500-1000 ppm) was taken in to a 15.0 mL graduated glass centrifuge tube, and hexanes added such that the total volume in the tube became 10.0 mL. To this mixture of additive and hexanes, asphaltenic stock solution (0.1 mL) was added. The test tube was then capped, shaken vigorously for about a minute or 40-60 times by hand and allowed to stand. The volume of any precipitated asphaltenes settled at the bottom of the tube was recorded at 10, 30, 60, 90 and 1440 (24 h) min intervals. When no additive was used, the volume of asphaltenes precipitated in the first 0.5-1 h was 0.4-0.5 mL (4-5%); in fact, it was important to initially adjust the concentration of the asphaltene stock in such a way that under these conditions of dilution with paraffinic solvents, a 4-5 vol% of asphaltenic precipitation occurred. When the additive was an effective dispersant of asphaltene, then no precipitate was formed up to 24 h (Rating = 2; good). In some cases, no precipitation was observed in over 24 h to several days (Rating = 2+; excellent). If the additive was not a dispersant, then an almost immediate precipitation of asphaltenes occurred (Rating = 0; poor).

Results for asphaltene dispersancy rating ("ADR") are displayed in Table 2, below. "Imidazoline 1" is 9-octadecenamide, N-{2-[2-(8-heptadecenyl) 4,5-dihydro-1H-imidazol-1-yl]ethyl}-; in "Imidazoline 2" the "R" attached to the amide group is a mixture of C₁₅-C₁₇ unsaturated alkyl groups and the group attached directly to the imidazoline ring is a 2-hydroxyethyl group; "Amine 1" is an isomeric mixture of C₁₀ to C₁₅ tertiary alkyl primary amines (PRIMENE 81-R; Rohm and Haas Company, Philadelphia, PA).

**Table 1**

| **Additive (mole ratio)** | **ADR** | **Active Ingredient, ppm** |
|---|---|---|
| L-tartaric acid/Imidazoline 1 (1:1) | 2 | 0.24 |
| L-tartaric acid/Imidazoline 1 (1:2) | 2+ | 0.49 |
| | | |
| oxalic acid/Imidazoline 1 (1:1) | 0 | 1000 |
| oxalic acid/Imidazoline 1 (1:2) | 2 | 0.246 |
| | | |
| citric acid/Imidazoline 1 (1:1) | 2 | 1.0 |
| citric acid/Imidazoline 1 (1:3) | 2 | 1.0 |
| | | |
| L-ascorbic acid/Imidazoline 1 (1:1) | 2 | 0.12 |
| | | |
| DL-malic acid/Imidazoline 1 (1:1) | 2 | 0.1 |
| | | |
| succinic acid/Imidazoline 1 (1:1) | 2 | 3.9 |
| | | |
| succinamic acid/Imidazoline 1 (1:1) | 2 | 250 |
| | | |
| decanoic acid/Imidazoline 1 (1:1) | 0 | 1000 |
| lauric acid/Imidazoline 1 (1:1) | 1 | 1000 |
| stearic acid/Imidazoline 1 (1:1) | 2 | 1000 |
| | | |
| salicylic acid/Imidazoline 1 (1:1) | 2 | 250 |
| | | |
| EDTA/Imidazoline 1 (1:2.5) | 2 | 3.125 |
| EDTA/Imidazoline 1 (1:0.1) | 2 | 6.25 |
| EDTA/Imidazoline 1 (1:4.4) | 2 | 3.12 |
| | | |
| L-tartaric acid/Imidazoline 2 (1:2) | 0 | 1000 |
| oxalic acid/imidazoline 2 (1:2) | 2+ | 125 |
| L-ascorbic acid/Imidazoline 2 (1:1) | 0 | 1000 |
| decanoic acid/Imidazoline 2 (1:1) | 0 | 1000 |
| DL-malic acid/Imidazoline 2 (1:1) | 0 | 1000 |
| succinic acid/Imidazoline 2 (1:1) | 0 | 1000 |
| EDTA/imidazoline 2 (1:4) | 0 | 1000 |
| hydrocinnamic acid/Imidazoline 2 (1:1) | 0 | 1000 |
| | | |
| Amine 1/L-ascorbic acid (2.2:1) | not soluble in Aromatic 150 | |
| Amine 1/L-tartaric acid (2.2:1) | no reaction | |
| Amine 1/oxalic acid (2.2:1) | no reaction | |
| Amine 1/citric acid (3.3:1) | 0 | at any concentration |
| Amine 1/EDTA (4.4:1) | 0 | at any concentration |
| | | |

## Claims

1. A composition comprising a reaction product of: (i) an imidazoline compound having two C₉-C₂₁ alkyl or C₉-C₂₁ alkenyl groups and (ii) an organic carboxylic acid having at least two carbon atoms and having at least one hydroxy group or at least one additional carboxyl group.

2. The composition of claim 1 in which the organic carboxylic acid has from two to ten carbon atoms.

3. The composition of claim 2 in which the imidazoline compound has two C₁₅-C₂₁ alkyl or C₁₅-C₂₁ alkenyl groups.

4. The composition of claim 3 in which the imidazoline compound has a structure as follows 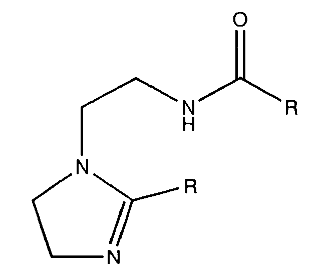 wherein R is a C₁₅-C₂₁ alkyl or C₁₅-C₂₁ alkenyl group.

5. The composition of claim 4 in which R is a C₁₅-C₁₇ alkyl or C₁₅-C₁₇ alkenyl group.

6. The composition of claim 5 further comprising a petroleum product containing asphaltenes.

7. The composition of claim 6 in which the organic carboxylic acid and the imidazoline compound are present in a mole ratio from 20:1 to 1:20, respectively.

8. The composition of claim 7 in which the organic carboxylic acid and the imidazoline compound are present in the petroleum product in a total amount from 0.05 ppm to 100 ppm.

9. The composition of claim 8 in which the petroleum product containing asphaltenes is heavy crude oil or residual fuel oil.

10. A method for dispersing asphaltenes in heavy crude oil or residual fuel oil; said method comprising adding to said heavy crude oil or residual fuel oil a composition comprising a reaction product of: (i) an imidazoline compound having two C₉-C₂₁ alkyl or C₉-C₂₁ alkenyl groups and (ii) an organic carboxylic acid.
